# FASCICULE DE BREVET EUROPEEN

(11) **EP 4 452 345 B1**
(45) Date de publication et mention de la délivrance du brevet: **01.10.2025**
(21) Numéro de dépôt: 22844101.0
(22) Date de dépôt: 22.12.2022
(51) Int. Cl.: A61L 27/12, A61L 27/36, A61L 27/38, C04B 35/447

(54) **MATÉRIAU DE RÉGÉNÉRATION OSSEUSE**
MATERIAL ZUR KNOCHENREGENERATION
BONE REGENERATION MATERIAL

(30) Priorité: 23.12.2021 BE 202106050
(43) Date de publication de la demande: 30.10.2024
(73) Titulaire: Wishbone, 4400 Flémalle (BE)
(72) Inventeur: ROMPEN, Eric, 4000 Liège (BE); LECLOUX, Geoffrey, 4050 Chaudfontaine (BE); LAMBERT, France, 4870 Trooz (BE); DORY, Emilie, 5300 Andenne (BE)
(74) Mandataire: Calysta NV
(86) Numéro de dépôt international: PCT/EP2022/087609
(87) Numéro de publication internationale: WO 2023/118505

(56) Documents cités:
- WO-A1-2015/049336
- WO-A1-2018/130686

## Description

La présente invention se rapporte à un matériau de régénération osseuse constitué essentiellement d'une phase solide d'hydroxyapatite d'origine naturelle macroporeuse.

La présente invention concerne en outre un procédé de fabrication du matériau de régénération osseuse constitué essentiellement d'une phase solide d'hydroxyapatite d'origine naturelle macroporeuse.

La présente invention se rapporte enfin à une méthode de réparation d'une défectuosité osseuse chez un patient, utilisant le matériau de régénération osseuse constitué essentiellement d'une phase solide d'hydroxyapatite d'origine naturelle macroporeuse.

Ce type de matériau de régénération osseuse est en particulier utilisé dans le cadre du traitement de détériorations osseuses et ce, dans différents domaines de la chirurgie réparatrice ou esthétique.

L'hydroxyapatite est un phosphate de calcium de formule Ca₅(PO₄)₃(OH) aux propriétés ostéoconductrices et constitue la composante minérale principale de l'os. En effet, l'hydroxyapatite appartient à la famille cristallographique des apatites qui sont des composés isomorphes possédant une même structure hexagonale.

Ce composé est largement utilisé comme biomatériau depuis de nombreuses années dans différentes spécialités médicales car les hydroxyapatites sont les phosphates de calcium cristallins les plus fréquents et les premiers constituants minéraux des os, de l'émail dentaire et de la dentine.

Par ailleurs, les hydroxyapatites et plus particulièrement les hydroxyapatites d'origine naturelle présentent de bonnes propriétés de biocompatibilité et d'adsorption spécifique des cellules ou des protéines, leur utilisation convient donc parfaitement dans le cadre d'une chirurgie reconstructrice, réparatrice ou esthétique de l'os, particulièrement orale.

Il est ainsi reconnu que l'hydroxyapatite d'origine naturelle (animale) présente des propriétés ostéoconductrices ainsi qu'une structure cristalline et une morphologie identique à celles d'un matériau osseux naturel chez l'humain, l'hydroxyapatite convenant parfaitement et étant actuellement utilisé comme implants, et en particulier comme implants intra-oraux, pour stimuler la reconstruction et régénération osseuse sur des sites osseux présentant des défauts ou des détériorations.

En outre, il a été identifié qu'un matériau de régénération osseuse constitué essentiellement d'une phase solide d'hydroxyapatite d'origine naturelle et nettoyée des substances organiques (protéines, prions, peptides, lipides) permettait d'obtenir une meilleure régénération osseuse en comparaison avec une phase solide d'hydroxyapatite synthétique. Il est en effet impératif que le matériau de régénération osseuse, lorsqu'il se trouve à l'état implanté chez le patient, soit épuré de toute trace de substances organiques de telle manière à favoriser son intégration dans l'organisme, son ostéointégration au site d'implantation, sa biocompatibilité et qu'il puisse interagir avec son environnement biologique en évitant les réactions de rejet indésirables.

La colonisation osseuse dépend des caractéristiques poreuses du matériau de régénération osseuse et de l'interconnexion entre ses macropores en nombre et en dimension. Ces interconnexions constituent des tunnels qui permettent le passage des cellules et l'afflux sanguin entre les pores, favorisant ainsi la formation d'un nouvel os.

Dans ce contexte, on connait de l'art antérieur le document US5417975 qui divulgue le produit BioOss^{®} qui est un matériau de régénération osseuse comprenant une phase solide d'hydroxyapatite d'origine naturelle présentant des nanopores, des micropores, et également des pores de diamètres supérieurs ou égaux à 50 µm.

On connait également de l'art antérieur les documents WO2015/049336 et WO2018/130686 qui divulguent également un matériau de régénération osseuse comprenant une phase solide d'hydroxyapatite d'origine naturelle présentant des pores de diamètres supérieurs ou égaux à 50 µm, de préférence des pores de diamètres compris entre 50 et 100 µm.

Malheureusement, bien que ces produits présentent de nombreux avantages, leurs propriétés mécaniques gagneraient à être améliorées.

La présente invention a pour but de pallier les inconvénients de l'état de la technique en procurant un matériau de régénération osseuse tel que mentionné ci-avant, caractérisé en ce que ladite phase solide d'hydroxyapatite est une phase solide cristalline d'hydroxyapatite dans laquelle les cristaux ont une taille comprise entre 20 et 120 nm, et présente une surface spécifique comprise entre 8 et 20 m²/g.

Le matériau de régénération osseuse selon la présente invention est de manière particulièrement avantageuse, obtenu après une étape de frittage à une température comprise entre 800°C et 1200°C, avantageusement comprise entre 800°C et 1150°C, de préférence entre 800°C et 1100°C, préférentiellement entre 800°C et 1050°C, avantageusement entre 800°C et 1000°C, de manière particulièrement avantageuse entre 800°C et 950°C, de préférence entre 800°C et 900°C, avantageusement entre 800°C et 850°C, par exemple entre 810°C et 830°C, comme 820°C.

Cette étape de frittage permet notamment de « souder » entre eux les cristaux de la phase solide d'hydroxyapatite d'origine naturelle, entrainant une très forte diminution des nanopores et des micropores, voire leur disparition complète, et l'augmentation de la taille des cristaux ainsi que la diminution de la surface spécifique. Cependant, la réduction massive, voire l'abolition des nanopores et des micropores du matériau de régénération osseuse est considérée comme néfaste puisque la vascularisation et la colonisation du matériau ne sera plus possible, si bien que la régénération osseuse ultérieure risque d'être incomplète ou de moins bonne qualité.

En effet, il est généralement reconnu que l'absence de microporosité dans un matériau de régénération osseuse est préjudiciable quant à la formation de la croissance osseuse et à l'ostéoconduction.

Contrairement à ce préjugé, l'étape de frittage selon la présente invention est particulièrement avantageuse car elle permet de préserver la topographie de surface du matériau de régénération, ce qui préserve le potentiel de régénération osseuse tout en renforçant sa résistance.

Par exemple, l'étape de frittage du matériau selon la présente invention, permet la formation d'éléments sensiblement sphériques à la surface du matériau.

Ainsi, le matériau selon la présente invention présente des éléments sensiblement sphériques/boules/pseudosphériques d'hydroxyapatite de taille (diamètre ou diamètre équivalent) comprise entre 150 et 350 nm, de préférence comprise entre 175 et 325 nm, préférentiellement comprise entre 200 et 300 nm. Les éléments sensiblement sphériques/boules/pseudosphériques forment ensemble une surface rugueuse du matériau de régénération osseuse selon la présente invention, contrairement à la mise en œuvre d'une étape de frittage dont la température est supérieure à 1200°C qui entraînerait une fonte de l'hydroxyapatite en surface d'un matériau, ce qui aurait pour conséquence de fournir une surface trop lisse, néfaste au potentiel de régénération osseuse.

De manière particulièrement surprenante, les inventeurs ont remarqué que le matériau de régénération osseuse selon la présente invention, fritté c'est-à-dire après une étape de frittage, et dans lequel les cristaux de la phase d'hydroxyapatite ont une taille comprise entre 20 et 120 nm, et présentant une surface spécifique comprise entre 8 et 20 m²/g, est aussi bien colonisé par le tissu osseux que les dispositifs de l'art antérieur tout en présentant une structure plus rigide, plus résistante et présentant une topographie de surface rugueuse.

En effet, le matériau de régénération osseuse selon la présente invention est destiné à être implanté pour la reconstruction et/ou la régénération d'un défaut osseux ou d'une détérioration, plus particulièrement dans le cadre dentaire, dans lequel les forces exercées par la mastication sont très importantes et répétitives dans le temps.

Il est donc particulièrement avantageux de pouvoir disposer du matériau de régénération osseuse selon la présente invention qui présente les mêmes caractéristiques et avantages que celles de l'art antérieur en termes de potentiel de régénération osseuse, d'ostéointégration, d'ostéoconduction tout en étant plus rigide, plus résistant et dont la rugosité de surface préserve, voire améliore, le potentiel de régénération osseuse.

Les revendications dépendantes se réfèrent à d'autres réalisations avantageuses.

Avantageusement, les cristaux de la phase solide cristalline d'hydroxyapatite du matériau de régénération osseuse selon la présente invention, ont une taille comprise entre 30 et 120 nm, de préférence comprise entre 40 et 100 nm, préférentiellement comprise entre 45 et 80 nm, de manière préférée comprise entre 50 et 80 nm, plus avantageusement comprise entre 50 et 60 nm.

Avantageusement, la surface spécifique de la phase solide d'hydroxyapatite du matériau de régénération osseuse selon la présente invention est comprise entre 10 et 20 m²/g, de préférence comprise entre 10 et 18 m²/g, de préférence entre 12 et 16 m²/g.

Cela présentant l'avantage de fournir un matériau de régénération osseuse selon la présente invention qui offre au moins les mêmes caractéristiques de potentiel de régénération osseuse que les dispositifs de l'art antérieur, tout en présentant une meilleure résistance et rigidité, particulièrement adapté au domaine dentaire dans lequel les contraintes mécaniques sont exigeantes.

De préférence, le matériau de régénération osseuse selon la présente invention présente une porosité comprise entre 70 et 85%, de préférence comprise entre 75 et 85%, préférentiellement comprise entre 80 et 85%.

Cela présentant l'avantage de fournir un matériau de régénération osseuse selon l'invention présentant un potentiel de formation osseuse significativement amélioré.

De préférence, le matériau de régénération osseuse selon la présente invention présente une distribution granulométrique d₁₀ comprise entre 350 et 500 µm, de préférence comprise entre 370 et 480 µm.

De préférence, le matériau de régénération osseuse selon la présente invention présente une distribution granulométrique d₅₀ comprise entre 500 et 800 µm, de préférence comprise entre 550 et 780 µm.

De préférence, le matériau de régénération osseuse selon la présente invention présente une distribution granulométrique d₉₀ comprise entre 850 et 1250 µm, de préférence comprise entre 850 et 1100 µm, préférentiellement comprise entre 850 et 1000 µm.

Le matériau de régénération osseuse selon la présente invention présentant une telle distribution granulométrique a l'avantage de présenter un volume poreux optimal permettant la régénération osseuse.

De manière avantageuse, le matériau de régénération osseuse selon la présente invention est enrichi par une deuxième phase solide synthétique de phosphate de calcium présentant un ratio molaire Ca/P compris entre 0,2 et 2, de préférence entre 0,3 et 1,8, préférentiellement compris entre 0,5 et 1,65, ladite deuxième phase solide synthétique ayant un produit de solubilité Ks supérieur au produit de solubilité Ks de ladite première phase d'hydroxyapatite solide d'origine naturelle.

Cela permettant de manière particulièrement avantageuse d'assurer un relargage adéquat de calcium (par exemple sous la forme d'ions libres extracellulaires Ca²⁺) et phosphore (par exemple sous la forme d'ions libres extracellulaires PO₄³⁻) dans l'environnement du site de régénération osseuse de telle sorte que ceux-ci puissent jouer le rôle de promoteurs de la repousse des tissus biologiques environnants en favorisant significativement la prolifération et la différentiation des cellules osseuses ainsi que la minéralisation.

Préférentiellement, le matériau de régénération osseuse selon la présente invention comprend au moins un agent thérapeutique choisi dans le groupe comprenant les antibiotiques, les antiviraux, les antiinflammatoires, les hormones telles que les stéroïdes, les facteurs de croissance tels que les BMP, les agents anti-rejets, les cellules souches, et leurs mélanges.

De manière avantageuse, le matériau de régénération osseuse selon la présente invention est un matériau stérile.

D'autres formes de réalisation du matériau de régénération osseuse selon la présente invention sont indiquées dans les revendications annexées.

La présente invention concerne également un procédé de fabrication du matériau de régénération osseuse selon la présente invention, comprenant :
- une mise en contact d'un matériau osseux, contenant de l'hydroxyapatite et des substances organiques avec une solution aqueuse d'extraction amenée à une température comprise entre 150°C et 300°C et à une pression comprise entre 1500 kPa et 3500 kPa, de manière à obtenir une première phase liquide contenant lesdites substances organiques et éventuellement des impuretés extraites dudit matériau osseux, et une deuxième phase d'hydroxyapatite solide,
- une séparation entre ladite phase liquide et ladite phase hydroxyapatite solide,
- un frittage (doux) de ladite phase d'hydroxyapatite solide séparée à une température comprise entre 800°C et 1200°C,
- ladite phase d'hydroxyapatite frittée formant ledit matériau de régénération osseuse.

Le procédé selon l'invention, permet de manière particulièrement surprenante et avantageuse, de fournir un matériau de régénération osseuse fritté qui présente au moins les mêmes caractéristiques de potentiel de régénération osseuse, d'ostéointégration, d'ostéoconduction que l'art antérieur, tout en étant plus rigide, plus résistant et dont la rugosité de surface préserve, voire améliore, le potentiel de régénération osseuse.

Il est notamment apparu que des conditions de frittage inférieures à 800°C ne permettaient pas une augmentation de la résistance mécanique du matériau, alors que des températures supérieures à 1200°C, voire supérieures à 900°C, entraînaient un impact négatif sur la topographie de surface et sur le potentiel de régénération osseuse du matériau tandis que des conditions de frittage comprises entre 800°C et 1200°C, voire inférieures à 900°C permettent d'obtenir un matériau de régénération osseuse solide, robuste, résistant et qui présente le potentiel de régénération osseuse recherché pour pouvoir être implanté chez le patient.

Avantageusement, le procédé selon l'invention comprend en outre série de tamisage sur une série de tamis, entre ladite étape de séparation et ladite étape de frittage, de ladite phase d'hydroxyapatite solide, de préférence ladite série de tamisage comprend au moins un premier tamisage sur un tamis de 1 mm et au moins un deuxième tamisage sur un tamis de 0,25 mm.

Par exemple, la phase d'hydroxyapatite solide après extraction, qui provient d'un matériau osseux, est déjà fragilisée par l'étape d'extraction et est déposée sur un set de tamis comprenant de bas en haut, un panier de collecte, un tamis de 0,25 mm et un tamis de 1 mm.

Encore plus avantageusement, la série de tamisage, entre l'étape de séparation et l'étape de frittage, du procédé selon l'invention, est une série de tamisage comprenant un ajout de billes métalliques sur ladite série de tamis, et une mise en mouvement desdites billes métalliques sur ladite série de tamis.

En effet, en ajoutant sur le set de tamis, des billes métalliques, qui sont mises en mouvement à l'aide d'un équipement, les billes vont davantage fragiliser la phase d'hydroxyapatite et le passage de cette dernière au travers des tamis en sera facilitée pour obtenir les particules dont la taille est souhaitée.

Avantageusement, la solution aqueuse d'extraction du procédé selon l'invention, est amenée à une température comprise entre 170°C et 280°C, de préférence entre 190°C et 260°C, préférentiellement entre 210°C et 240°C, avantageusement entre 220°C et 230°C.

De préférence, la solution aqueuse d'extraction du procédé selon l'invention, est amenée à une pression comprise entre 2000 et 3500 kPa, de préférence entre 2500 et 3500 kPa, préférentiellement entre 3000 et 3500 kPa, avantageusement entre 3200 et 3500 kPa, voire entre 3400 et 3500 kPa.

En effet, l'étape d'extraction super critique du procédé selon l'invention dans des conditions de température comprise entre 220 et 230°C et de pression comprise entre 3200 et 3500 kPa, présente les meilleurs résultats pour obtenir une phase hydroxyapatite solide pure, laquelle est nettoyée des substances organiques (protéines, prions, peptides, lipides) diminuant ainsi les réactions de rejet indésirables qui pourraient survenir ultérieurement.

La durée de l'étape d'extraction super critique est avantageusement adaptée en fonction de la quantité de matériau osseux. Avantageusement, l'étape de frittage du procédé selon la présente invention a lieu pendant une durée comprise entre 40 minutes et 4 heures, de préférence pendant une durée comprise entre 1 et 3 heures, préférentiellement entre 1 et 2 heures, par exemple entre 1 et 1,5 heures.

En outre l'étape de frittage comprend une sous-étape de montée en température couplée à une étape de plateau de chauffe ; cette étape de frittage dure au total par exemple 1h 20 minutes.

De préférence, l'étape de frittage du procédé selon la présente invention a lieu à une température comprise entre 800°C et 1150°C, de préférence entre 800°C et 1100°C, préférentiellement entre 800°C et 1050°C, avantageusement entre 800°C et 1000°C, de manière particulièrement avantageuse entre 800°C et 950°C, de préférence entre 800°C et 900°C, avantageusement entre 800°C et 850°C, par exemple entre 810°C et 830°C.

De préférence, l'étape de montée en température de l'étape de frittage permet d'atteindre la température comprise entre 800°C et 1150°C, avantageusement entre 800°C et 850°C, par exemple entre 810°C et 830°C, pendant une durée comprise entre 20mn et 2h, de préférence entre 20mn et 1h, préférentiellement entre 20mn et 45mn, avantageusement entre 25 et 35mn.

Par exemple l'étape de montée en température permet d'atteindre la température de frittage de 820°C en 30mn.

De préférence, pendant l'étape de montée en température, l'incrément de température en fonction du temps est substantiellement linéaire.

Ceci permet une étape de montée en température qui ne soit pas trop longue, et qui soit reproductible.

En effet, les inventeurs ont constaté qu'une étape de montée en température présentant des durées relativement longues, surtout à des températures supérieures à 600°C, affectait déjà l'os ; ceci n'est pas nécessairement pénalisant, mais doit être pris en compte pour la détermination de la durée de l'étape de plateau à la température comprise entre 800°C et 1200°C, de préférence entre 800°C et 1150°C, avantageusement entre 800°C et 850°C, par exemple entre 810°C et 830°C.

En outre, l'étape de plateau de chauffe de l'étape de frittage, est pratiquée à une température comprise entre 800°C et 1200°C, avantageusement entre 800°C et 850°C, par exemple entre 810°C et 830°C, pendant une durée comprise entre 20mn et 2h, de préférence entre 30mn et 1h30, préférentiellement entre 45mn et 1h, avantageusement entre 46mn et 58mn.

Par exemple l'étape de plateau de chauffe à une température de frittage de 820°C a lieu pendant une durée comprise entre 45mn et 58mn.

En outre, l'étape de frittage du procédé selon l'invention présente une certaine flexibilité quant à la température et à la durée de frittage. Cependant, le paramètre de la température de l'étape du plateau de chauffe dans l'étape de frittage est le plus important et gagne à être davantage contrôlé.

Avantageusement, le procédé selon la présente invention comprend en outre entre ladite étape de tamisage et ladite étape de frittage, une étape de traitement aux peroxydes de ladite phase d'hydroxyapatite solide, de préférence une étape de traitement à l'eau oxygénée.

De préférence, le procédé selon l'invention comprend en outre une entre ladite étape de traitement aux peroxydes et ladite étape de frittage, une étape de séchage (doux).

De manière avantageuse, le procédé selon la présente invention comprend en outre une étape d'enrichissement du matériau de régénération osseuse en calcium et en phosphore, par au moins un premier et au moins un deuxième trempage distincts se succédant l'un l'autre dans un ordre quelconque, ledit au moins un premier trempage ayant lieu dans une première solution comprenant du calcium à une concentration de 1M et ledit au moins un deuxième trempage ayant lieu dans une deuxième solution comprenant du phosphore à une concentration de 0,5M.

Avantageusement, le premier trempage de l'étape d'enrichissement du procédé selon l'invention, a lieu dans une première solution de Ca(NO₃)₂.4H₂O, de CaCl₂.2H₂O, de CaSO₄.2H₂O ou de CaCO₃.

Avantageusement, le deuxième trempage de l'étape d'enrichissement du procédé selon l'invention, a lieu dans une deuxième solution de Na₃PO₄, de Na₂HPO₄, de NaH₂PO₄.H₂O, de K₃PO₄, de K₂HPO₄, de KH₂PO₄, de K₂HPO₄, de (NH₄)₃PO₄, de (NH₄)₂HPO₄ ou de NH₄H₂PO₄.

De préférence, le procédé selon l'invention comprend en outre une étape de stérilisation du matériau de régénération osseuse et/ou du matériau de régénération osseuse enrichi, de préférence une étape de stérilisation par ionisation.

D'autres formes de réalisation du procédé de fabrication selon la présente invention sont indiquées dans les revendications annexées.

D'autres caractéristiques, détails et avantages de l'invention ressortiront de la description donnée ci-après, à titre non limitatif et en faisant référence aux dessins et aux exemples.

### Exemples.-

La figure 1 illustre une image obtenue par microscopie à balayage électronique MEB de la structure d'un matériau de régénération osseuse selon l'art antérieur, fritté à une température supérieure à 1200°C.
Les figures 2A et 2B illustrent une image obtenue par microscopie à balayage électronique MEB de la structure du matériau de régénération osseuse selon l'invention, fritté à une température de 820°C, respectivement à un grossissement de 5.000 fois et 10.000 fois.

### Exemple 1 - Matériau de régénération osseuse selon la présente invention

On a réalisé un matériau de régénération osseuse selon la présente invention qui est constitué essentiellement d'une phase solide d'hydroxyapatite. Dans le cas présent, le matériau a été stérilisé.

On a réalisé une série d'analyses comprenant notamment la détermination de la composition de la phase solide, de la taille des cristaux, de la porosité volumétrique, de la taille des particules, de la surface spécifique, pour 3 échantillons du matériau de régénération osseuse selon la présente invention.

Les échantillons 1, 2 et 3 présentent tous les trois une composition de la phase solide de 100% d'hydroxyapatite. C'est-à-dire, le matériau de régénération osseuse selon la présente invention est constitué essentiellement d'une phase solide d'hydroxyapatite.

L'échantillon 1 présente une taille de cristaux de 54,6 nm, l'échantillon 2 de 54,2 nm et l'échantillon 3 de 54,4 nm. La taille moyenne des cristaux du matériau osseux selon la présente invention est donc de 54,4 nm.

Concernant la porosité volumétrique, l'échantillon 1 présente une porosité de 82,3%, l'échantillon 2 de 82,1% et l'échantillon 3 de 82,5%. La porosité volumétrique moyenne du matériau de régénération osseuse selon la présente invention est ainsi de 82,3%.

Concernant la taille de particules, l'échantillon 1 présente une distribution granulométrique d₁₀ de 381 µm, d₅₀ de 553 µm et d₉₀ de 877 µm. L'échantillon 2 présente une distribution granulométrique d₁₀ de 452 µm, d₅₀ de 782 µm et d₉₀ de 1243 µm. L'échantillon 3 présente une distribution granulométrique d₁₀ de 474 µm, d₅₀ de 756 µm et d₉₀ de 1115 µm. La distribution granulométrique moyenne d₁₀ du matériau de régénération osseuse selon la présente invention est donc de 436 µm, la d₅₀ de 697 µm et la d₉₀ de 1079 µm.

Les échantillons 1, 2 et 3 présentent tous les trois une surface spécifique de 16 m²/g et donc une surface spécifique moyenne de 16 m²/g.

En outre, le matériau de régénération osseuse selon la présente invention est obtenu de manière particulièrement avantageuse après une étape de frittage à une température de 820°C pendant une durée comprise entre 45mn et 60mn de plateau de chauffe, permettant d'obtenir un matériau présentant une structure plus rigide, plus résistante et présentant une topographie de surface rugueuse, améliorant le potentiel de régénération osseuse.

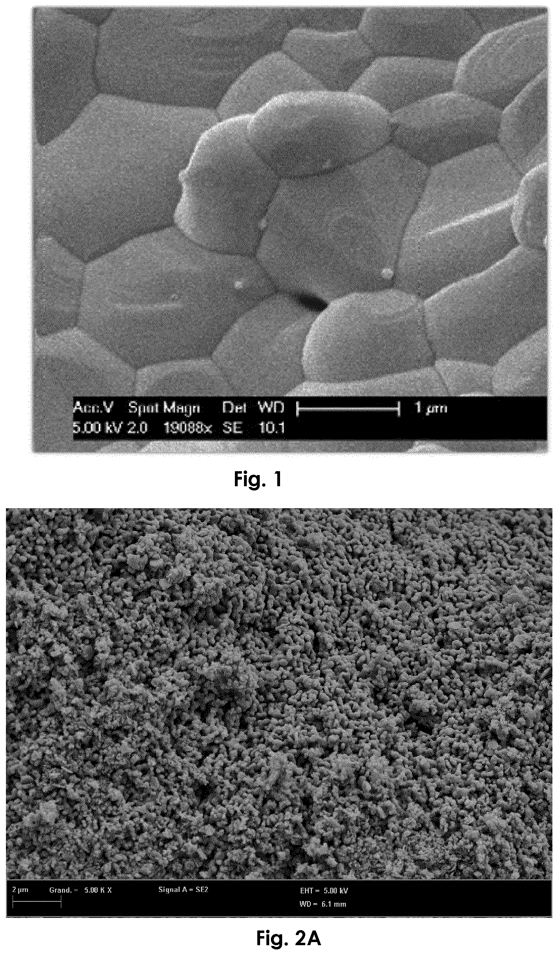

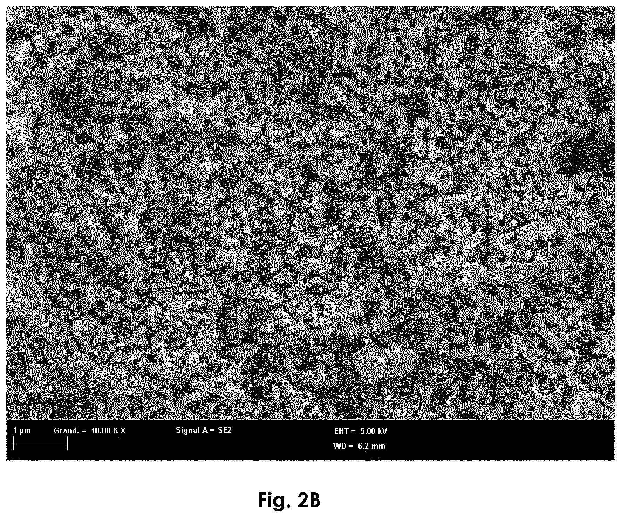

En effet, comme cela est illustré dans les figures 2A et 2B, le matériau selon la présente invention présente des éléments sensiblement sphériques/boules/pseudosphériques d'hydroxyapatite de taille (diamètre ou diamètre équivalent) comprise entre 150 et 350 nm, de préférence comprise entre 175 et 325 nm, préférentiellement comprise entre 200 et 300 nm, qui forment ensemble une surface rugueuse. Contrairement au matériau de régénération osseuse illustré à la figure 1 qui a été fritté à une température supérieure à 1200°C et qui présente une surface lisse, défavorable au potentiel de régénération osseuse.

### Exemple 2 - Préparation du matériau de régénération osseuse selon la présente invention.

On a préparé un lot de matériau de régénération osseuse selon la présente invention en collectant des os de bovins puis en effectuant une découpe des os de bovins afin de former un matériau osseux.

Le matériau osseux contenant de l'hydroxyapatite et des substances organiques a été mis en contact avec une solution aqueuse d'extraction dans des conditions de température et de pression supercritiques, par exemple entre 220°C et 230°C et à une pression comprise entre 3200 et 3500 kPa, afin d'obtenir une première phase liquide contenant les substances organiques et impuretés extraites et une deuxième phase d' hydroxyapatite solide, ces deux phases ont ensuite été séparées pour conserver la phase d'hydroxyapatite solide.

La phase d'hydroxyapatite solide fragilisée par l'étape d'extraction supercritique, est placée sur un premier tamis de 1mm pour effectuer un premier tamisage, puis sur un deuxième tamis de 0,25mm pour effectuer un deuxième tamisage. Avantageusement, des billes métalliques sont placées sur les tamis avec la phase d'hydroxyapatite solide pour faciliter les cassures et le tamisage. Les étapes de tamisage peuvent avoir lieu une ou plusieurs fois, par exemple 2 ou 3 fois.

La phase d'hydroxyapatite solide après tamisage est recueillie et soumise à une étape de frittage à une température comprise entre 800 et 1200°C, idéalement à une température de 820°C pendant une étape de plateau de chauffe à 820°C comprise entre 45mn et 60mn.

La phase d'hydroxyapatite solide frittée forme ainsi le matériau de régénération osseuse selon la présente invention, lequel présente une structure plus rigide, est plus résistant et présente également une topographie de surface rugueuse, améliorant ainsi le potentiel de régénération osseuse.

En outre, la phase d'hydroxyapatite solide frittée formant le matériau selon l'invention peut également être rincée et/ou enrichie en calcium et en phosphore par des trempages distincts, puis stérilisé, de préférence par ionisation.

## Revendications

1. Matériau de régénération osseuse constitué essentiellement d'une phase solide d'hydroxyapatite d'origine naturelle macroporeuse présentant des pores de diamètres supérieurs ou égaux à 50 µm, de préférence des pores de diamètres compris entre 50 et 100 µm, **caractérisé en ce que** ladite phase solide d' hydroxyapatite est une phase solide cristalline d'hydroxyapatite dans laquelle les cristaux ont une taille comprise entre 20 et 120 nm, de préférence comprise entre 30 et 120 nm, de préférence comprise entre 40 et 100 nm, préférentiellement comprise entre 45 et 80 nm, de manière préférée comprise entre 50 et 80 nm, plus avantageusement comprise entre 50 et 60 nm, et ladite phase solide d'hydroxyapatite présente une surface spécifique comprise entre 8 et 20 m²/g, de préférence comprise entre 10 et 20 m²/g, de préférence comprise entre 10 et 18 m²/g, de préférence entre 12 et 16 m²/g.

2. Matériau de régénération osseuse selon la revendication 1, lequel présente une porosité comprise entre 70 et 85%, de préférence comprise entre 75 et 85%, préférentiellement comprise entre 80 et 85%.

3. Matériau de régénération osseuse selon l'une quelconque des revendications 1 ou 2, lequel présente une distribution granulométrique d₁₀ comprise entre 350 et 500 µm, de préférence comprise entre 370 et 480 µm, et/ou lequel présente une distribution granulométrique d₅₀ comprise entre 500 et 800 µm, de préférence comprise entre 550 et 780 µm, et/ou lequel présente une distribution granulométrique d₉₀ comprise entre 850 et 1250 µm, de préférence comprise entre 850 et 1100 µm, préférentiellement comprise entre 850 et 1000 µm.

4. Matériau de régénération osseuse selon l'une quelconque des revendications 1 à 3, lequel est enrichi par une deuxième phase solide synthétique de phosphate de calcium présentant un ratio molaire Ca/P compris entre 0,2 et 2, de préférence entre 0,3 et 1,8, préférentiellement compris entre 0,5 et 1,65, ladite deuxième phase solide synthétique ayant un produit de solubilité Ks supérieur au produit de solubilité Ks de ladite première phase d'hydroxyapatite solide d'origine naturelle.

5. Matériau de régénération osseuse selon l'une quelconque des revendications 1 à 4, comprenant au moins un agent thérapeutique choisi dans le groupe comprenant les antibiotiques, les antiviraux, les antiinflammatoires, les hormones telles que les stéroïdes, les facteurs de croissance tels que les BMP, les agents anti-rejets, les cellules souches, et leurs mélanges.

6. Matériau de régénération osseuse selon l'une quelconque des revendications 1 à 5, lequel est un matériau stérile.

7. Procédé de fabrication d'un matériau de régénération osseuse selon l'une quelconque des revendications 1 à 6, comprenant :
- une mise en contact d'un matériau osseux, contenant de l'hydroxyapatite et des substances organiques avec une solution aqueuse d'extraction amenée à une température comprise entre 150°C et 300°C, de préférence comprise entre 170°C et 280°C, de préférence entre 190°C et 260°C, préférentiellement entre 210°C et 240°C, avantageusement entre 220°C et 230°C, et à une pression comprise entre 1500 kPa et 3500 kPa, préférentiellement comprise entre 2000 et 3500 kPa, de préférence entre 2500 et 3500 kPa, préférentiellement entre 3000 et 3500 kPa, avantageusement entre 3200 et 3500 kPa, de manière à obtenir une première phase liquide contenant lesdites substances organiques et éventuellement des impuretés extraites dudit matériau osseux, et une deuxième phase d'hydroxyapatite solide,
- une séparation entre ladite phase liquide et ladite phase hydroxyapatite solide,
- avantageusement comprenant en outre une série de tamisage sur une série de tamis, entre ladite étape de séparation et ladite étape de frittage, de ladite phase d'hydroxyapatite solide, de préférence ladite série de tamisage comprend au moins un premier tamisage sur un tamis de 1 mm et au moins un deuxième tamisage sur un tamis de 0,25 mm, plus préférentiellement dans lequel ladite série de tamisage, entre ladite étape de séparation et ladite étape de frittage, est une série de tamisage comprenant un ajout de billes métalliques sur ladite série de tamis, et une mise en mouvement desdites billes métalliques sur ladite série de tamis,
- un frittage (doux) de ladite phase d'hydroxyapatite solide séparée à une température comprise entre 800°C et 1200°C, de manière préférentielle comprise entre 800°C et 1150°C, de préférence entre 800°C et 1 100°C, préférentiellement entre 800°C et 1050°C, avantageusement entre 800°C et 1000°C, de manière particulièrement avantageuse entre 800°C et 950°C, de préférence entre 800°C et 900°C, avantageusement entre 800°C et 850°C, par exemple entre 810°C et 830°C,
- ladite phase d'hydroxyapatite frittée formant ledit matériau de régénération osseuse.

8. Procédé selon la revendication 7, dans lequel ladite étape de frittage a lieu pendant une durée comprise entre 40mn et 4 heures, de préférence pendant une durée comprise entre 1 et 3 heures, préférentiellement entre 1 et 2 heures, par exemple entre 1 et 1,5 heures.

9. Procédé selon l'une quelconque des revendications 7 ou 8, comprenant en outre entre ladite étape de tamisage et ladite étape de frittage, une étape de traitement aux peroxydes de ladite phase d'hydroxyapatite solide, de préférence une étape de traitement à l'eau oxygénée, plus préférentiellement comprenant en outre une entre ladite étape de traitement aux peroxydes et ladite étape de frittage, une étape de séchage.

10. Procédé selon l'une quelconque des revendications 7 à 9, comprenant en outre une étape additionnelle de rinçage de ladite phase d'hydroxyapatite frittée.

11. Procédé selon l'une quelconque des revendications 7 à 10, comprenant en outre une étape d'enrichissement du matériau de régénération osseuse en calcium et en phosphore, par au moins un premier et au moins un deuxième trempage distincts se succédant l'un l'autre dans un ordre quelconque, ledit au moins un premier trempage ayant lieu dans une première solution comprenant du calcium à une concentration de 1M et ledit au moins un deuxième trempage ayant lieu dans une deuxième solution comprenant du phosphore à une concentration de 0,5M, de préférence ledit premier trempage de ladite étape d'enrichissement, a lieu dans une première solution de Ca(NO₃)₂.4H₂O, de CaCl₂.2H₂O, de CaSO₄.2H₂O ou de CaCO₃, et/ou ledit deuxième trempage de ladite étape d'enrichissement, a lieu dans une deuxième solution de Na₃PO₄, de Na₂HPO₄, de NaH₂PO₄.H₂O, de K₃PO₄, de K₂HPO₄, de KH₂PO₄, de K₂HPO₄, de (NH₄)₃PO₄, de (NH₄)₂HPO₄ ou de NH₄H₂PO₄.

12. Procédé selon l'une quelconque des revendications 7 à 11, comprenant en outre une étape de stérilisation du matériau de régénération osseuse et/ou du matériau de régénération osseuse enrichi, de préférence une étape de stérilisation par ionisation.

## Patentansprüche

1. Knochenregenerationsmaterial, im Wesentlichen bestehend aus einer makroporösen festen Phase von Hydroxylapatit natürlichen Ursprungs, das Poren mit einem Durchmesser größer als oder gleich wie 50 µm, vorzugsweise Poren mit einem Durchmesser zwischen 50 und 100 µm, aufweist, **dadurch gekennzeichnet, dass** die feste Phase von Hydroxylapatit eine kristalline feste Phase von Hydroxylapatit ist, wobei die Kristalle eine Größe zwischen 20 und 120 nm, vorzugsweise zwischen 30 und 120 nm, vorzugsweise zwischen 40 und 100 nm, bevorzugterweise zwischen 45 und 80 nm, bevorzugt zwischen 50 und 80 nm, vorteilhafterweise zwischen 50 und 60 nm, aufweisen, und die feste Phase aus Hydroxylapatit eine spezifische Oberfläche zwischen 8 und 20 m²/g, vorzugsweise zwischen 10 und 20 m²/g, vorzugsweise zwischen 10 und 18 m²/g, vorzugsweise zwischen 12 und 16 m²/g aufweist.

2. Knochenregenerationsmaterial nach Anspruch 1, die eine Porosität zwischen 70 und 85 %, vorzugsweise zwischen 75 und 85 %, bevorzugterweise zwischen 80 und 85% umfasst.

3. Knochenregenerationsmaterial nach einem der Ansprüche 1 oder 2, die eine Korngrößenverteilung d₁₀ zwischen 350 und 500 µm, vorzugsweise zwischen 370 und 480 µm, aufweist, und/oder die eine Korngrößenverteilung d₅₀ zwischen 500 und 800 µm, vorzugsweise zwischen 550 und 780 µm, aufweist, und/oder die eine Korngrößenverteilung d₉₀ zwischen 850 und 1250 µm, vorzugsweise zwischen 850 und 1100 µm, bevorzugterweise zwischen 850 und 1000 µm, aufweist.

4. Knochenregenerationsmaterial nach einem der Ansprüche 1 bis 3, die mit einer zweiten synthetischen festen Phase von Calciumphosphat angereichert ist, das ein Molverhältnis Ca/P zwischen 0,2 und 2, vorzugsweise zwischen 0,3 und 1,8, bevorzugterweise zwischen 0,5 und 1,65 aufweist, wobei die zweite synthetische feste Phase ein Löslichkeitsprodukt Ks größer als das Löslichkeitsprodukt Ks der ersten natürlich vorkommenden festen Hydroxylapatitphase aufweist.

5. Knochenregenerationsmaterial nach einem der Ansprüche 1 bis 4, umfassend mindestens ein therapeutisches Mittel, das ausgewählt ist aus der Gruppe, bestehend aus Antibiotika, antiviralen Mitteln, entzündungshemmenden Mitteln, Hormonen, wie beispielsweise Steroide, Wachstumsfaktoren, wie beispielsweise BMPs, abstoßungverhindernden Mitteln, Stammzellen und Gemischen davon.

6. Knochenregenerationsmaterial nach einem der Ansprüche 1 bis 5, das ein steriles Material ist.

7. Verfahren zur Herstellung eines Knochenregenerationsmaterials nach einem der Ansprüche 1 bis 6, umfassend:
- Inkontaktbringen eines Knochenmaterials, das Hydroxylapatit und organische Substanzen enthält, mit einer wässrigen Extraktionslösung, die auf eine Temperatur zwischen 150 °C und 300 °C, vorzugsweise zwischen 170 °C und 280 °C, vorzugsweise zwischen 190 °C und 260 °C, gebracht wird, bevorzugterweise zwischen 210 °C und 240 °C, vorteilhafterweise zwischen 220 °C und 230 °C, und auf einen Druck zwischen 1500 kPa und 3500 kPa, bevorzugterweise zwischen 2000 und 3500 kPa, vorzugsweise zwischen 2500 und 3500 kPa, bevorzugterweise zwischen 3000 und 3500 kPa, vorteilhafterweise zwischen 3200 und 3500 kPa, gebracht wird, um eine erste flüssige Phase, die die organischen Substanzen und optional Verunreinigungen enthält, die aus dem genannten Knochenmaterial extrahiert werden, und eine zweite Phase aus festem Hydroxylapatit zu erlangen,
- eine Trennung zwischen der flüssigen Phase und der festen Hydroxylapatitphase,
- vorzugsweise ferner umfassend eine Reihe von Siebvorgängen der festen Hydroxylapatitphase auf einer Reihe von Sieben zwischen dem Trennschritt und dem Sinterschritt, wobei die Reihe von Siebvorgängen vorzugsweise mindestens ein erstes Sieben auf einem 1 mm Sieb und mindestens ein zweites Sieben auf einem 0,25 mm Sieb umfasst, bevorzugter, wobei die Reihe von Siebvorgängen zwischen dem Trennschritt und dem Sinterschritt eine Reihe von Siebvorgängen ist, die ein Hinzufügen von Metallkugeln auf die Reihe von Sieben und ein Bewegen der Metallkugeln auf der Reihe von Sieben umfasst,
- ein (weiches) Sintern der getrennten festen Hydroxylapatitphase bei einer Temperatur zwischen 800 °C und 1200 °C, bevorzugt zwischen 800 °C und 1150 °C, vorzugsweise zwischen 800 °C und 1100 °C, bevorzugterweise zwischen 800 °C und 1050 °C, vorteilhafterweise zwischen 800 °C und 1000 °C, besonders vorteilhafterweise zwischen 800 °C und 950 °C, vorzugsweise zwischen 800 °C und 900 °C, vorteilhafterweise zwischen 800 °C und 850 °C, zum Beispiel zwischen 810 °C und 830 °C,
- wobei die gesinterte Hydroxylapatitphase das Knochenregenerationsmaterial bildet.

8. Verfahren nach Anspruch 7, wobei der Sinterschritt über eine Dauer zwischen 40 Minuten und 4 Stunden erfolgt, vorzugsweise über eine Dauer zwischen 1 und 3 Stunden, bevorzugterweise zwischen 1 und 2 Stunden, beispielsweise zwischen 1 und 1,5 Stunden.

9. Verfahren nach einem der Ansprüche 7 oder 8, ferner umfassend zwischen dem Siebschritt und dem Sinterschritt einen Behandlungsschritt mit Peroxid der festen Hydroxylapatitphase, vorzugsweise einen Behandlungsschritt mit Wasserstoffperoxid, bevorzugter ferner umfassend einen Trocknungsschritt zwischen dem Behandlungsschritt mit Peroxid und dem Sinterschritt.

10. Verfahren nach einem der Ansprüche 7 bis 9, ferner umfassend einen zusätzlichen Schritt eines Spülens der gesinterten Hydroxylapatitphase.

11. Verfahren nach einem der Ansprüche 7 bis 10, ferner umfassend einen Anreicherungsschritt des Anreicherns des Knochenregenerationsmaterials mit Kalzium und Phosphor durch mindestens ein erstes und mindestens ein zweites separates Einweichen, die in beliebiger Reihenfolge aufeinander folgen, wobei das mindestens eine erste Einweichen in einer ersten Lösung erfolgt, umfassend Calcium in einer Konzentration von 1M, und das mindestens eine zweite Einweichen in einer zweiten Lösung erfolgt, umfassend Phosphor in einer Konzentration von 0,5M, vorzugsweise das erste Einweichen des Anreicherungsschritts in einer ersten Lösung von Ca(NO₃) ₂.4H₂O CaCl₂.2H₂O CaSO₄.2H₂O oder CaCO₃ erfolgt, und/oder das zweite Einweichen des Anreicherungsschritts in einer zweiten Lösung von Na₃PO₄, Na₂HPO₄, NaH₂PO₄.H₂O, K₃PO₄, K₂HPO₄, KH₂PO₄, K₂HPO₄, (NH₄)₃PO₄, (NH₄)₂HPO₄ oder NH₄H₂PO₄ erfolgt.

12. Verfahren nach einem der Ansprüche 7 bis 11, ferner umfassend einen Sterilisationsschritt des Knochenregenerationsmaterials und/oder des angereicherten Knochenregenerationsmaterials, vorzugsweise einen Sterilisationsschritt durch Ionisation.

## Claims

1. Bone regeneration material consisting essentially of a solid phase of macroporous natural hydroxyapatite having pores with diameters greater than or equal to 50 µm, preferably pores with diameters of between 50 and 100 µm, **characterised in that** said solid phase of hydroxyapatite is a crystalline solid phase of hydroxyapatite in which the crystals have a size of between 20 and 120 nm, preferably between 30 and 120 nm, preferably between 40 and 100 nm, preferably between 45 and 80 nm, preferably between 50 and 80 nm, more advantageously between 50 and 60 nm, and said solid phase of hydroxyapatite has a specific surface area of between 8 and 20 m²/g, preferably between 10 and 20 m²/g, preferably between 10 and 18 m²/g, of preferably between 12 and 16 m²/g.

2. Bone regeneration material according to claim 1, which has a porosity of between 70 and 85%, preferably between 75 and 85%, preferably between 80 and 85%.

3. Bone regeneration material according to any one of claims 1 or 2, which has a particle size distribution d₁₀ of between 350 and 500 µm, preferably between 370 and 480 µm, and/or which has a particle size distribution d₅₀ of between 500 and 800 µm, preferably between 550 and 780 µm, and/or which has a particle size distribution d₉₀ of between 850 and 1250 µm, preferably between 850 and 1100 µm, preferably between 850 and 1000 µm.

4. Bone regeneration material according to any one of claims 1 to 3, which is enriched with a second synthetic solid phase of calcium phosphate having a Ca/P molar ratio of between 0.2 and 2, preferably between 0.3 and 1.8, preferably between 0.5 and 1.65, said second synthetic solid phase having a solubility product Ks greater than the solubility product Ks of said first phase of solid hydroxyapatite of natural origin.

5. Bone regeneration material according to any one of claims 1 to 4, comprising at least one therapeutic agent selected from the group comprising antibiotics, antivirals, anti-inflammatories, hormones such as steroids, growth factors such as BMPs, anti-rejection agents, stem cells, and mixtures thereof.

6. Bone regeneration material according to any one of claims 1 to 5, which is a sterile material.

7. A method of manufacturing a bone regeneration material according to any one of claims 1 to 6, comprising:
- bringing a bone material, containing hydroxyapatite and organic substances, into contact with an aqueous extraction solution brought to a temperature of between 150°C and 300°C, preferably between 170°C and 280°C, preferably between 190°C and 260°C, preferably between 210°C and 240°C, advantageously between 220°C and 230°C, and at a pressure of between 1500 kPa and 3500 kPa, preferably between 2000 and 3500 kPa, preferably between 2500 and 3500 kPa, preferably between 3000 and 3500 kPa, advantageously between 3200 and 3500 kPa, so as to obtain a first liquid phase containing said organic substances and optionally impurities extracted from said bone material, and a second phase of solid hydroxyapatite,
- a separation between said liquid phase and said solid hydroxyapatite phase,
- advantageously further comprising a sieving series on a series of sieves, between said separation step and said sintering step, of said solid hydroxyapatite phase, preferably said series of sieving comprises at least a first sieving on a 1 mm sieve and at least a second sieving on a 0.25 mm sieve, more preferably in which said series of sieving, between said separation step and said sintering step, is a sieving series comprising an addition of metal balls on said series of sieves, and a setting in motion of said metal balls on said series of sieves,
- (soft) sintering of said separated solid hydroxyapatite phase at a temperature of between 800°C and 1200°C, preferably between 800°C and 1150°C, preferably between 800°C and 1100°C, preferably between 800°C and 1050°C, advantageously between 800°C and 1000°C, particularly advantageously between 800°C and 950°C, preferably between 800°C and 900°C, advantageously between 800°C and 850°C, for example between 810°C and 830°C,
- said sintered hydroxyapatite phase forming said bone regeneration material.

8. Method from claim 7, wherein said sintering step takes place for a period of between 40 minutes and 4 hours, preferably for a period of between 1 and 3 hours, preferably between 1 and 2 hours, for example between 1 and 1.5 hours.

9. Method from any one of claims 7 or 8, further comprising, between said sieving step and said sintering step, a peroxide treatment step of said solid hydroxyapatite phase, preferably a hydrogen peroxide treatment step, more preferably further comprising a drying step between said peroxide treatment step and said sintering step.

10. Method from any one of claims 7 to 9, further comprising an additional step of rinsing said sintered hydroxyapatite phase.

11. Method according to any one of claims 7 to 10, further comprising a step of enriching the bone regeneration material with calcium and phosphorus, by at least one first and at least one second separate soaking following one another in any order, said at least one first soaking taking place in a first solution comprising calcium at a concentration of 1M and said at least one second soaking taking place in a second solution comprising phosphorus at a concentration of 0.5M, preferably said first soaking of said enrichment step takes place in a first solution of Ca(NO₃)₂.4H₂O, CaCl₂.2H₂O, CaSO₄.2H₂O or CaCO₃, and/or said second soaking of said enrichment step takes place in a second solution of Na₃PO₄, Na₂HPO₄, NaH₂PO₄.H₂O, K₃PO₄, K₂HPO₄, KH₂PO₄, K₂HPO₄, (NH₄)₃PO₄, (NH₄)₂HPO₄ or NH₄H₂PO₄.

12. Method according to any one of claims 7 to 11, further comprising a step of sterilising the bone regeneration material and/or the enriched bone regeneration material, preferably a step of sterilising by ionisation.
